# EUROPEAN PATENT SPECIFICATION

(11) **EP 3 238 723 B1**
(45) Date of publication and mention of the grant of the patent: **05.02.2020**
(21) Application number: 15873009.3
(22) Date of filing: 21.12.2015
(51) Int. Cl.: A61K 31/551, A61P 27/02, C07D 487/04

(54) **DRUG FOR TREATING DISORDERS OF CORNEAL EPITHELIUM**
ARZNEIMITTEL ZUR BEHANDLUNG VON ERKRANKUNGEN DES HORNHAUTEPITHELS
MÉDICAMENT POUR LE TRAITEMENT DE TROUBLES DE L'ÉPITHÉLIUM CORNÉEN

(30) Priority: 22.12.2014 JP 2014258383
(43) Date of publication of application: 01.11.2017
(73) Proprietor: EA Pharma Co., Ltd., Tokyo 104-0042 (JP)
(72) Inventor: MIZUSHIMA, Tohru, Tokyo 105-0022 (JP); IWATA, Hiroshi, Chuo-ku, Tokyo 1040042 (JP)
(74) Representative: Mewburn Ellis LLP
(86) International application number: PCT/JP2015/085704
(87) International publication number: WO 2016/104441

(56) References cited:
- EP-A1- 1 346 993
- WO-A1-02/44180
- WO-A1-2008/136392
- JP-A- 2007 537 260
- US-A1- 2007 207 522

## Description

### Field of the Invention

The present invention relates to an agent for the treatment of corneal epithelium disorders.

### Background of the Invention

Corneal epithelium disorders are diseases associated with eye discomfort or visual dysfunction caused by various factors, such as dry eye, inappropriate use of contact lenses, antiseptic agents included in eye drops, and autoimmune diseases including Sjögren's syndrome. Among these, particularly the number of dry eye patients is said to be about 22 millions in Japan, and the number of patients who suffer from corneal epithelium disorders tends to increase. Further, the prevalence rate increases with aging.

In regard to dry eye, it is considered that the lacrimal fluid becomes hyperosmotic as a result of reduced secretion and increased evaporation of the lacrimal fluid, inflammatory response and cell injury occur in the cornea, and the lacrimal fluid layer is unstabilized, so that as a result, the onset of corneal epithelium disorders proceeds (Non-Patent Literature 1) .

Regarding the method for the treatment of dry eye currently used, for example, lacrimal fluid replacement therapy using sodium hyaluronate, diquafosol sodium (acceleration of mucin secretion), and rebamipide (acceleration of mucin production); use of spectacles or goggles; and lacrimal opening plug method are used.

Patent Literature 2 discloses the use of the glycoprotein lacritin, and fragments, derivatives, and homologs thereof, and nucleic acid sequences encoding that protein. The invention relates to the use of lacritin as a growth factor for human corneal epithelial cells and other select epithelial cells . The invention also relates to the use of lacritin in promoting ocular cell survival in response to an insult or injury, in protecting against ocular inflammation, and in promoting ocular wound repair.

Non-Patent Literature 2 and 3 disclose compounds of general formula (I) given below, wherein B represents a benzene ring, -X- and -Y- each represent -NH-, -Z-represents -CH₂-, -W-represents -NH- and -A(R²)(R³)(R⁴) represents a phenyl group, 4-bromophenyl group, 4-hydroxyphenyl group, 4-methoxyphenyl group, 2-hydroxyphenyl group, 3,4-dimethoxyphenyl group or 3-methoxy-4-hydroxyphenyl group.

Non-Patent Literature 4 and 5 disclose compounds of general formula (I) given below, wherein B represents a benzene ring, -X- represents -NH-, -Z-represents -CR⁶R⁷-, -W-represents -NH- and R⁶ and R⁷ each represent a methyl group.

Non-Patent Literature 6 discloses a compound of general formula (I) given below, wherein B represents a benzene ring, -X- represents -NH-, -Z-represents -CO-, -W- represents -NR¹- and R¹ represents p-tolyl group.

Non-Patent Literature 7 discloses compounds of general formula (I) given below, wherein B represents a benzene ring, -X- represents -NH-, -Y- represents -S-, -Z-represents -CR⁶R⁷-, W represents -NH- and R⁶ and R⁷ each represent a methyl group.

Non-Patent Literature 8 also discloses compounds of general formula (I) given below, wherein B represents a benzene ring, -X- represents -NH-, -Y- represents -S-, -Z-represents -CH2- and -W- represents -O-.

### Citation List

### Patent Literature

Patent Literature 1: WO 2002/044180 A
Patent Literature 2: US 2007/207522 A1

### Non Patent Literature

Non-Patent Literature 1: Website homepage of Japanese Ophthalmological Society
Non-Patent Literature 2: Khim.-Farm. Zh., 25 (11), (1991)
Non-Patent Literature 3: Pharmaceutical Chemical Journal, 25 (11), 768 (1991)
Non-Patent Literature 4: Journal of the Pharmaceutical Society of Japan, 715-20 (1986)
Non-Patent Literature 5: Chem. Pharm. Bull., 3724-9 (1984)
Non-Patent Literature 6: Synthesis, 937-8 (1987)
Non-Patent Literature 7: Journal of the Pharmaceutical Society of Japan, 1004-8 (1984)
Non-Patent Literature 8: Journal of Organic Chemistry, 4367-70 (1983)

### Summary of Invention

### Technical Problem

However, in regard to the conventional method for treatment of dry eye, the main purpose lies in replacement of lacrimal fluid or prevention of drying, and the method for the treatment does not act directly on the corneal epithelial cells. Thus, it is hard to say that a sufficient therapeutic effect is obtained therefrom. Furthermore, under the circumstances, sufficient treatment is not achieved for corneal epithelium disorders that occur as a result of diabetes mellitus or long-term use of eye drops.

Therefore, an object of the present invention is to provide a new agent for the treatment of corneal epithelium disorders, which acts directly on corneal epithelial cells.

### Solution to Problem

Thus, the inventors of the present invention conducted extensive investigations in search of compounds having protective action on corneal epithelial cells with inflammation or cell injury, in connection with dry eye, for example. As a result, totally unexpectedly, the inventors found that some lactam compounds that are known to have sugar transport enhancement action and hypoglycemic action, strongly suppress a decrease in the survival rate of corneal epithelial cells caused by hyperosmotic stress, and this action is based on the action of corneal epithelial cells for suppressing apoptosis under hyperosmotic stress; and that since the lactam compounds prevent corneal epithelial cell injury and also increase the amount of lacrimal fluid or mucin, the lactam compounds are useful as agents for the treatment of corneal epithelium disorders caused by dry eye, for example. Thus, the inventors completed the present invention.

That is, the present invention provides the following items [1] to [6].
[1] A compound of Formula (1) or a salt thereof, for use in the treatment of a corneal epithelium disorder: 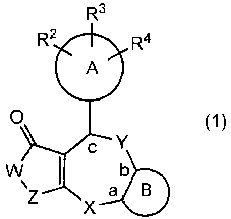
   wherein A is a benzene ring,
   R², R³, and R⁴, which may be identical or different, each independently are a hydrogen atom, a halogen atom, a hydroxy group, an alkyl group, a mercapto group, an alkoxy group, an alkylthio group, an alkylsulfonyl group, an acyl group, an acyloxy group, an amino group, an alkylamino group, a carboxy group, an alkoxycarbonyl group, a carbamoyl group, a nitro group, a cyano group, a trifluoromethyl group, an alkenyl group which may have a substituent, an alkynyl group which may have a substituent, an aryl group which may have a substituent, a heteroaryl group which may have a substituent, a benzyloxy group which may have a substituent, an aryloxy group which may have a substituent, a heteroaryloxy group which may have a substituent, an arylamino group which may have a substituent, an arylvinyl group which may have a substituent, or an arylethynyl group which may have a substituent; B is a cyclohexane ring;
   X is -NH-; Y is -NR⁵-; and Z is -CH₂-
   (wherein R⁵ is a lower alkyl group which may have a substituent, an acyl group which may have a substituent, an alkoxycarbonyl group which may have a substituent, a carbamoyl group which may have a substituent, or a sulfonyl group which may have a substituent);
   -W- is -NR¹-, (wherein
   R¹ is a hydrogen atom, a lower alkyl group which may have a substituent, or an aryl group which may have a substituent) ;
   and a, b, and c each represent the position of a carbon atom; provided that:
      (i) said optional substituent is selected from the group consisting
      of a halogen atom, a hydroxy group, an alkyl group, a mercapto group, an alkoxy group, an alkylthio group, an alkylsulfonyl group, an acyl group, an acyloxy group, an amino group, an alkylamino group, a carboxy group, an alkoxycarbonyl group, a carbamoyl group, a nitro group, a cyano group, a trifluoromethyl group, an aryl group, and a heteroaryl group;
[2] The compound according to [1] or a salt thereof, wherein R¹ is H, and R⁵ is an acyl group which may have a substituent.
[3] The compound according to any one of [1] to [2] or a salt thereof, wherein the compound of Formula (1) isacompound of the following Formula (1a): 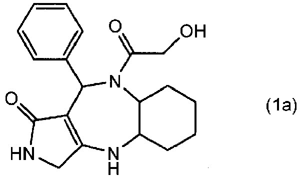
[4] The compound according to any one of [1] to [3] or a salt thereof, wherein the compound is an eye drop.
[5] The compound according to any one of [1] to [4] or a salt thereof, wherein the corneal epithelium disorder is dry eye.
[6] A compound of Formula (1) or a salt thereof, for medical use in suppressing corneal epithelial cell apoptosis: 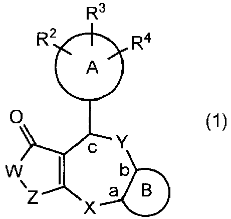
   wherein A is a benzene ring;
   R², R³, and R⁴, which may be identical or different, each independently are a hydrogen atom, a halogen atom, a hydroxy group, an alkyl group, a mercapto group, an alkoxy group, an alkylthio group, an alkylsulfonyl group, an acyl group, an acyloxy group, an amino group, an alkylamino group, a carboxy group, an alkoxycarbonyl group, a carbamoyl group, a nitro group, a cyano group, a trifluoromethyl group, an alkenyl group which may have a substituent, an alkynyl group which may have a substituent, an aryl group which may have a substituent, a heteroaryl group which may have a substituent, a benzyloxy group which may have a substituent, an aryloxy group which may have a substituent, a heteroaryloxy group which may have a substituent, an arylamino group which may have a substituent, an arylvinyl group which may have a substituent, or an arylethynyl group which may have a substituent; B is a cyclohexane ring;
   X is -NH-; Y is -NR⁵-; and Z is -CH₂-
   (wherein R⁵ is a lower alkyl group which may have a substituent, an acyl group which may have a substituent, an alkoxycarbonyl group which may have a substituent, a carbamoyl group which may have a substituent, or a sulfonyl group which may have a substituent) ;
   -W- is -NR¹-, (wherein
   R¹ is a hydrogen atom, a lower alkyl group which may have a substituent, or an aryl group which may have a substituent);
   and a, b, and c each represent the position of a carbon atom; provided that:
      (i) said optional substituent is selected from the group consisting
      of a halogen atom, a hydroxy group, an alkyl group, a mercapto group, an alkoxy group, an alkylthio group, an alkylsulfonyl group, an acyl group, an acyloxy group, an amino group, an alkylamino group, a carboxy group, an alkoxycarbonyl group, a carbamoyl group, a nitro group, a cyano group, a trifluoromethyl group, an aryl group, and a heteroaryl group.

### Advantageous Effects of Invention

The compound for the treatment of a corneal epithelium disorder of the present invention suppresses apoptosis of corneal epithelial cells and also has an effect of increasing lacrimal fluid and an effect of increasing mucin. The compound for the treatment of a corneal epithelium disorder improves symptoms of the dry eye and also enables radical cure of the dry eye. Brief Description of Drawings

Fig. 1 illustrates a decrease in the survival rate of corneal epithelial cells caused by hyperosmotic stress (addition of NaCl).
Fig. 2 illustrates the effects of Compound (1) of the present invention on the decrease in the survival rate of corneal epithelial cells caused by hyperosmotic stress (addition of NaCl) .
Fig. 3 illustrates the effects of Compound (1) of the present invention on the decrease in the survival rate of corneal epithelial cells caused by hyperosmotic stress (addition of glucose).
Fig. 4 illustrates the effects of Compound (1) of the present invention on the decrease in the survival rate of corneal epithelial cells caused by ethanol.
Fig. 5 illustrates the effects of Compound (1) of the present invention on the hyperosmotic stress (addition of NaCl)-dependent apoptosis of corneal epithelial cells.
Fig. 6 illustrates the effects of Compound (1) of the present invention on the amount of lacrimal fluid in a dry eye model rat.
Fig. 7 illustrates the effects of Compound (1) of the present invention on the injury of corneal surface in a dry eye model rat.
Fig. 8 illustrates the effects of Compound (1) of the present invention on the expression of a mucin-producing gene in corneal epithelial cells.

### Description of Embodiments

The active ingredient for the treatment of a corneal epithelium disorder of the present invention is a compound of Formula (1) or a salt thereof. This compound is a compound described in WO 2002/044180 A, and is known to be useful as an agent for the treatment of diabetes mellitus. However, nothing is known about the action of the compound on corneal epithelium disorders.

In Formula (1), A is a benzene ring.

R², R³, and R⁴, which may be identical or different, each independently are a hydrogen atom, a halogen atom, a hydroxy group, an alkyl group, a mercapto group, an alkoxy group, an alkylthio group, an alkylsulfonyl group, an acyl group, an acyloxy group, an amino group, an alkylamino group, a carboxy group, an alkoxycarbonyl group, a carbamoyl group, a nitro group, a cyano group, a trifluoromethyl group, an alkenyl group which may have a substituent, an alkynyl group which may have a substituent, an aryl group which may have a substituent, a heteroaryl group which may have a substituent, a benzyloxy group which may have a substituent, an aryloxy group which may have a substituent, a heteroaryloxy group which may have a substituent, an arylamino group which may have a substituent, an arylvinyl group which may have a substituent, or an arylethynyl group which may have a substituent. However, a hydrogen atom, a halogen atom, a hydroxy group, an alkyl group, an alkoxy group, an alkylthio group, a carboxy group, or an alkoxycarbonyl group are preferred. Furthermore, a case in which R², R³, and R⁴ all are a hydrogen atom is particularly preferred.

B is a cyclohexane ring.

X is -NH-, Y is NR⁵-, and Z is -CH₂-. Here,
R⁵ is a lower alkyl group which may have a substituent, an acyl group which may have a substituent, an alkoxycarbonyl group which may have a substituent, a carbamoyl group which may have a substituent, or a sulfonyl group which may have a substituent. However, a lower alkyl group, or an acyl group which may have s substituent is preferred; an alkanoyl group which may have a substituent is more preferred; a C₂-C₄ alkanoyl group which may have a substituent is even more preferred; and an acetyl group which may have a substituent is still more preferred.

(i) The substituent described above is selected from the group consisting of a halogen atom, a hydroxy group, an alkyl group, a mercapto group, an alkoxy group, an alkylthio group, an alkylsulfonyl group, an acyl group, an acyloxy group, an amino group, an alkylamino group, a carboxy group, an alkoxycarbonyl group, a carbamoyl group, a nitro group, a cyano group, a trifluoromethyl group, an aryl group, and a heteroaryl group. Among these, a halogen atom, a hydroxy group, a mercapto group, an alkoxy group, or an alkylthio group is preferred; a hydroxy group or an alkoxy group is more preferred; and a hydroxy group is even more preferred.

W is -NR¹-.
Here, R¹ is preferably a hydrogen atom or a lower alkyl group, and more preferably a hydrogen atom.

A compound in which
R¹ is H; and R⁵ is an acyl group which may have a substituent, is even more preferred.

Among compounds of Formula (1), a compound of the following Formula (1a) is more preferred:

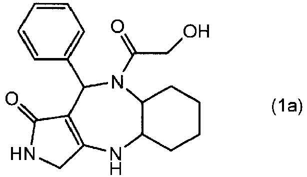

Furthermore, a compound of the following Formula (1aa) [(1R,8R,10R)-9-(2-hydroxyacetyl)-8-phenyl-2,5,9-triazatricy clo[8.4.0.0^{3,7}]tetradeca-3(7)-en-6-one] is particularly preferred.

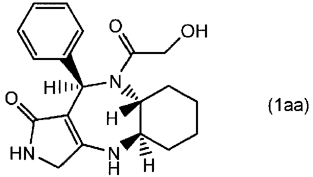

Examples of a salt of the compound of Formula (1) (hereinafter, may be referred to as Compound (1) of the present invention) include basic salts such as an ammonium salt, an alkali metal salt (for example, sodium salt, potassium salt), and an alkaline earth metal salt (for example, calcium salt, magnesium salt); and acid addition salts such as hydrochloride, sulfate, nitrate, acetate, lactate, and citrate.

Furthermore, Compound (1) of the present invention has an asymmetric carbon atom and thus has optical isomers. Therefore, optically active substances are also included therein. Moreover, Compound (1) of the present invention or a salt thereof may also be in the form of a hydrate or a solvate.

Compound (1) of the present invention or a salt thereof can be produced according to the method described in WO 2002/044180 A.

As illustrated in the Examples described below, Compound (1) of the present invention or a salt thereof is useful as an agent for the treatment of corneal epithelium disorders because the compound suppresses a decrease in the survival rate of corneal epithelial cells caused by hyperosmotic stress, suppresses the hyperosmotic stress-dependent apoptosis of corneal epithelial cells, and suppresses corneal injury. Corneal epithelium disorders include corneal epithelium disorders involved in infectious diseases caused by inappropriate use of contact lenses, and chemistry of eye drop antiseptic agents, autoimmune diseases such as Sjögren's syndrome, diabetes mellitus, and dry eye. Since Compound (1) or a salt thereof has an effect of increasing the amount of lacrimal fluid and the amount of mucin, Compound (1) or a salt thereof is useful particularly as an agent for the treatment of dry eye.

Examples of the dosage form for the compound for the treatment of corneal epithelium disorders of the present invention include an eye drop, an injectable preparation, an oral preparation (tablets, a granular preparation, a powder, and capsules), an ointment, and a cream. Among these, an eye drop is particularly preferred. In order to produce the compound into the form of these pharmaceutical compositions, the compound can be formulated together with a pharmaceutically acceptable carrier. Examples of such a carrier include lactose, glucose, D-mannitol, starch, crystalline cellulose, calcium carbonate, kaolin, starch, gelatin, hydroxypropyl cellulose, hydroxypropylmethyl cellulose, polyvinylpyrrolidone, ethanol, carboxymethyl cellulose, carboxymethyl cellulose calcium salt, magnesium stearate, talc, acetyl cellulose, white sugar, titanium oxide, benzoic acid, paraoxybenzoic acid ester, sodium dehydroacetate, gumarabic, tragacanth, methyl cellulose, eggyolk, surfactants, white sugar, simple syrup, citric acid, distilledwater, ethanol, glycerin, propylene glycol, macrogol, sodium hydrogen phosphate, sodium dihydrogen phosphate, sodium phosphate, glucose, sodium chloride, phenol, thimerosal, para-oxybenzoic acid ester, and sodium hydrogen sulfite . These carriers are used in appropriate mixtures with Compound (1) of the present invention in accordance with the dosage form.

When the pharmaceutical composition of the present invention is produced into an eye drop, for example, sodium hyaluronate, diquafosol sodium, and rebamipide can also be incorporated in addition to Compound (1) of the present invention or a salt thereof.

Furthermore, the content of the active ingredient of the present invention in the pharmaceutical composition preparation of the present invention greatly depends on the form of the preparation and is not particularly limited. However, the content is usually 0.01% to 100% by weight, and preferably 1% to 100% by weight, with respect to the total amount of the composition.

The dosage of the compound for corneal epithelium disorders of the present invention may depend on the symptoms and age of the patient in need of administration, and the method for administration; however, the dosage is preferably from 0.01 to 100 mg/kg/day.

### EXAMPLES

Next, the present invention will be described in more detail by way of Examples.

### Example 1 (Suppressive effect on cell injury under hyperosmotic conditions)

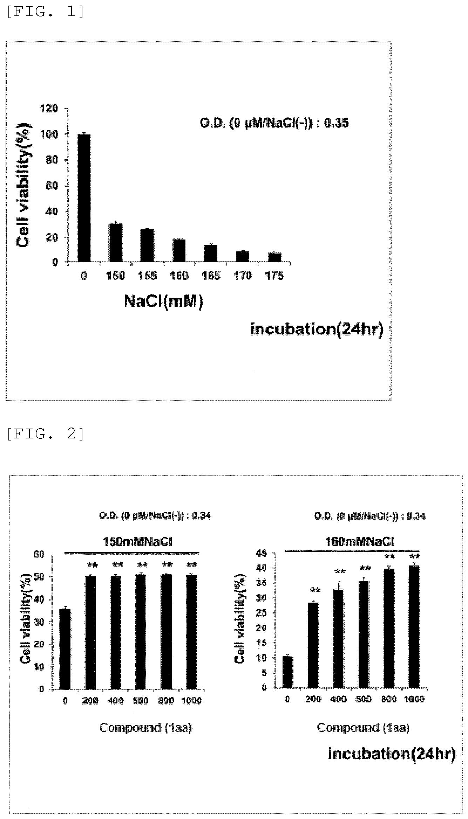

HCE cells (human corneal epithelial cells) were cultured for 24 hours in a hyperosmotic medium containing a compound of Formula (1aa) (hereinafter, Compound (1aa)) (as a hydrate). The medium was adjusted to fulfill the hyperosmotic conditions using 150 mM NaCl and 160 mM NaCl, or 320 mM glucose. The number of live cells was measured by the MTT method, and relative values of light absorbance with respect to the light absorbance of the control (isotonic pressure conditions) were calculated. The results are presented in Fig. 1 to Fig. 3. The value is expressed as average value ± S.D. (n = 3), **P < 0.01.

As can be seen from Fig. 1 to Fig. 3, Compound (1aa) noticeably suppressed the decrease in the survival rate of corneal epithelial cells caused by the hyperosmotic conditions.

### Example 2 (Suppressive effect on cell injury caused by ethanol)

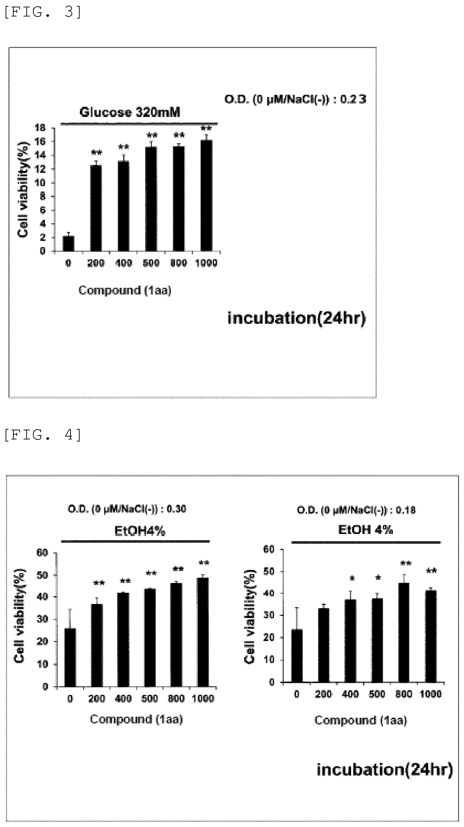

HCE cells (human corneal epithelial cells) were cultured in a medium containing Compound (laa) and 4 v/v% ethanol. The number of live cells was measured by the MTT method, and relative values of light absorbance with respect to the light absorbance of the control (absence of ethanol) were calculated. The results are presented in Fig. 4. The value is expressed as average value ± S.D. (n = 3), *P < 0.05; and **P < 0.01.

As can be seen from Fig. 4, Compound (laa) noticeably suppressed the decrease in the survival rate of corneal epithelial cells caused by ethanol.

### Example 3 (Suppressive effect on apoptosis under hyperosmotic conditions)

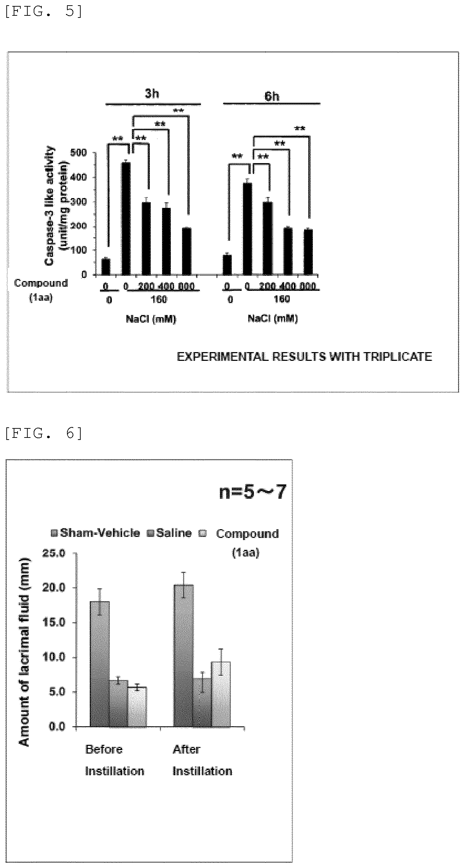

HCE cells were cultured in a hyperosmotic medium containing Compound (laa) and 160 mM NaCl. After culturing for 3 hours and 6 hours, caspase-3-like activity, which is a marker for apoptosis, was measured using a fluorescent peptide substrate, and the results are presented in Fig. 5. The value is expressed as average value ± S.D. (triplicate), **P < 0.01, and the term n.s. means "not significant".

As can be seen from Fig. 5, Compound (laa) noticeably suppressed hyperosmotic stress-dependent apoptosis of corneal epithelial cells.

### Example 4 (Therapeutic effect on corneal surface injury in rat)

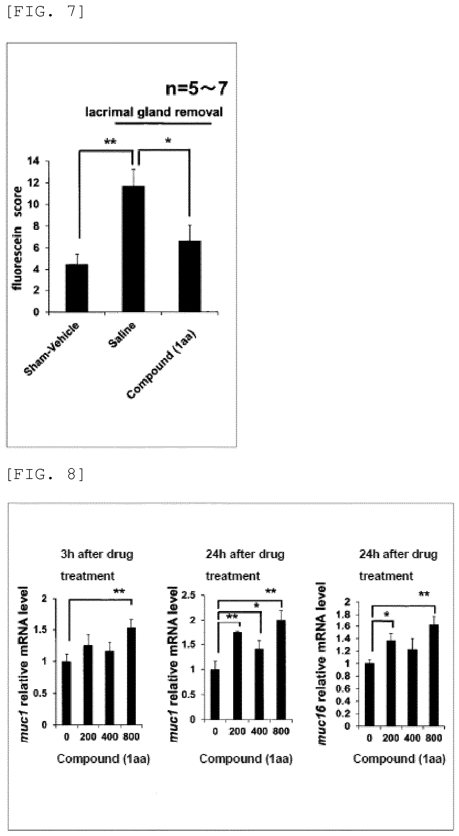

The lacrimal glands of a rat were removed, and a dry eye model was produced. After 1 to 5 weeks from the removal of lacrimal glands, 5 µL of an aqueous solution containing Compound (laa) (0.05 w/v%, 1.5 mM) was administered to the rat three times a day. The amount of lacrimal fluid was measured by a cotton thread test, and the results are presented in Fig. 6. An image of cornea stained with fluorescein is presented in Fig. 7. The scores of fluorescein were calculated, and the results are presented in Fig. 7. The value is expressed as average value ± S.E.M., *P < 0.05; and **P < 0.01.

As can be seen from Fig. 6 and Fig. 7, Compound (1aa) suppressed injury of the corneal surface by increasing the amount of lacrimal fluid in the dry eye model rat.

### Example 5 (Change in amount of expression of messenger RNA (effect on mucin expression))

HCE cells were cultured in a medium containing Compound (1aa). The amounts of expression of muc1 and muc16 messenger RNAs after culturing for 3 hours and 24 hours were measured by a real time RT-PCR method. Relative values of the amount of expression with respect to the amount of expression of actin messenger RNA were calculated, and the results are presented in Fig. 8. The value is expressed as average value ± S.D. (triplicate), *P < 0.05; and **P < 0.01.

As can be seen from Fig. 8, it became clear that Compound (laa) increases the amount of expression of mucin in corneal epithelial cells.

## Claims

1. A compound of Formula (1) or a salt thereof, for use in the treatment of a corneal epithelium disorder: 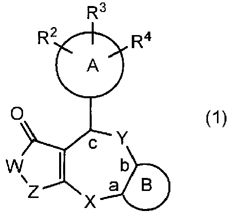 wherein A is a benzene ring; R², R³, and R⁴, which may be identical or different, each independently are a hydrogen atom, a halogen atom, a hydroxy group, an alkyl group, a mercapto group, an alkoxy group, an alkylthio group, an alkylsulfonyl group, an acyl group, an acyloxy group, an amino group, an alkylamino group, a carboxy group, an alkoxycarbonyl group, a carbamoyl group, a nitro group, a cyano group, a trifluoromethyl group, an alkenyl group which may have a substituent, an alkynyl group which may have a substituent, an aryl group which may have a substituent, a heteroaryl group which may have a substituent, a benzyloxy group which may have a substituent, an aryloxy group which may have a substituent, a heteroaryloxy group which may have a substituent, an arylamino group which may have a substituent, an arylvinyl group which may have a substituent, or an arylethynyl group which may have a substituent; B is a cyclohexane ring; X is -NH-; Y is -NR⁵-; and Z is -CH₂- (wherein R⁵ is an acyl group which may have a substituent, a lower alkyl group which may have a substituent, an alkoxycarbonyl group which may have a substituent, a carbamoyl group which may have a substituent, or a sulfonyl group which may have a substituent) ; W is -NR¹-; (wherein R¹ is a hydrogen atom, a lower alkyl group which may have a substituent, or an aryl group which may have a substituent) ; and a, b, and c each represent the position of a carbon atom; provided that:
(i) said optional substituent is selected from the group consisting of a halogen atom, a hydroxy group, an alkyl group, a mercapto group, an alkoxy group, an alkylthio group, an alkylsulfonyl group, an acyl group, an acyloxy group, an amino group, an alkylamino group, a carboxy group, an alkoxycarbonyl group, a carbamoyl group, a nitro group, a cyano group, a trifluoromethyl group, an aryl group, and a heteroaryl group.

2. The compound for use according to claim 1 or a salt thereof, wherein R¹ is H, and R⁵ is an acyl group which may have a substituent.

3. The compound for use according to any one of claims 1 to 2 or a salt thereof, wherein the compound of Formula (1) is a compound of the following Formula (1a):

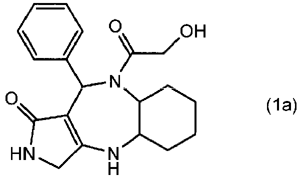

4. The compound for use according to any one of claims 1 to 3 or a salt thereof, wherein the compound is administered as a pharmaceutical composition which is an eye drop.

5. The compound for use according to any one of claims 1 to 4 or a salt thereof, wherein the corneal epithelium disorder is dry eye.

6. A compound of Formula (1) or a salt thereof, for medical use in suppressing corneal epithelial cell apoptosis: 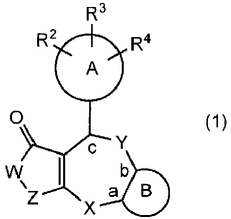 wherein A is a benzene ring; R², R³, and R⁴, which may be identical or different, each independently are a hydrogen atom, a halogen atom, a hydroxy group, an alkyl group, a mercapto group, an alkoxy group, an alkylthio group, an alkylsulfonyl group, an acyl group, an acyloxy group, an amino group, an alkylamino group, a carboxy group, an alkoxycarbonyl group, a carbamoyl group, a nitro group, a cyano group, a trifluoromethyl group, an alkenyl group which may have a substituent, an alkynyl group which may have a substituent, an aryl group which may have a substituent, a heteroaryl group which may have a substituent, a benzyloxy group which may have a substituent, an aryloxy group which may have a substituent, a heteroaryloxy group which may have a substituent, an arylamino group which may have a substituent, an arylvinyl group which may have a substituent, or an arylethynyl group which may have a substituent; B is a cyclohexane ring; X is -NH-; Y is -NR⁵-; and Z is -CH₂- (wherein R⁵ is an acyl group which may have a substituent, a lower alkyl group which may have a substituent, an alkoxycarbonyl group which may have a substituent, a carbamoyl group which may have a substituent, or a sulfonyl group which may have a substituent) ; W is -NR¹-; (wherein R¹ is a hydrogen atom, a lower alkyl group which may have a substituent, or an aryl group which may have a substituent) ; and a, b, and c each represent the position of a carbon atom; provided that:
(i) said optional substituent is selected from the group consisting of a halogen atom, a hydroxy group, an alkyl group, a mercapto group, an alkoxy group, an alkylthio group, an alkylsulfonyl group, an acyl group, an acyloxy group, an amino group, an alkylamino group, a carboxy group, an alkoxycarbonyl group, a carbamoyl group, a nitro group, a cyano group, a trifluoromethyl group, an aryl group, and a heteroaryl group.-

## Patentansprüche

1. Verbindung nach Formel (1) oder ein Salz davon zur Verwendung zur Behandlung einer Hornhautepithelstörung: 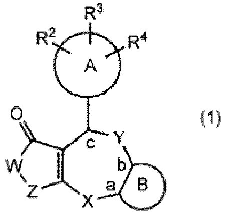 worin A ein Benzolring ist; R², R³ und R⁴, die gleich oder verschieden sein können, jeweils unabhängig Folgendes sind: ein Wasserstoffatom, ein Halogenatom, eine Hydroxygruppe, eine Alkylgruppe, eine Mercaptogruppe, eine Alkoxygruppe, eine Alkylthiogruppe, eine Alkylsulfonylgruppe, eine Acylgruppe, eine Acyloxygruppe, eine Aminogruppe, eine Alkylaminogruppe, eine Carboxygruppe, eine Alkoxycarbonylgruppe, eine Carbamoylgruppe, eine Nitrogruppe, eine Cyanogruppe, eine Trifluormethylgruppe, eine Alkenylgruppe, die gegebenenfalls einen Substituenten aufweist, eine Alkinylgruppe, die gegebenenfalls einen Substituenten aufweisen, eine Arylgruppe, die gegebenenfalls einen Substituenten aufweist, eine Heteroarylgruppe, die gegebenenfalls einen Substituenten aufweist, eine Benzyloxygruppe, die gegebenenfalls einen Substituenten aufweist, eine Aryloxygruppe, die gegebenenfalls einen Substituenten aufweist, eine Heteroaryloxygruppe, die gegebenenfalls einen Substituenten aufweist, eine Arylaminogruppe, die gegebenenfalls einen Substituenten aufweist, eine Arylvinylgruppe, die gegebenenfalls einen Substituenten aufweist, oder eine Arylethinylgruppe, die gegebenenfalls einen Substituenten aufweist; B ein Cyclohexanring ist; X -NH- ist; Y -NR⁵- ist und Z -CH₂- ist (worin R⁵ eine Acylgruppe, die gegebenenfalls einen Substituenten aufweist, eine Niederalkylgruppe, die gegebenenfalls einen Substituenten aufweist, eine Alkoxycarbonylgruppe, die gegebenenfalls einen Substituenten aufweist, eine Carbamoylgruppe, die gegebenenfalls einen Substituenten aufweist, oder eine Sulfonylgruppe, die gegebenenfalls einen Substituenten aufweist, ist); W -NR¹- ist (worin R¹ ein Wasserstoffatom, eine Niederalkylgruppe, die gegebenenfalls einen Substituenten aufweist, oder eine Arylgruppe, die gegebenenfalls einen Substituenten aufweist, ist) und a, b und c jeweils die Position eines Kohlenstoffatoms anzeigen; mit der Maßgabe, dass:
(i) der optionale Substituent aus der aus folgenden bestehenden Gruppe ausgewählt ist: Halogenatom, Hydroxygruppe, Alkylgruppe, Mercaptogruppe, Alkoxygruppe, Alkylthiogruppe, Alkylsulfonylgruppe, Acylgruppe, Acyloxygruppe, Aminogruppe, Alkylaminogruppe, Carboxygruppe, Alkoxycarbonylgruppe, Carbamoylgruppe, Nitrogruppe, Cyanogruppe, Trifluormethylgruppe, Arylgruppe und Heteroarylgruppe.

2. Verbindung zur Verwendung nach Anspruch 1 oder ein Salz davon, wobei R¹ H ist und R⁵ eine Acylgruppe ist, die gegebenenfalls einen Substituenten aufweist.

3. Verbindung zur Verwendung nach einem der Ansprüche 1 bis 2 oder ein Salz davon, wobei die Verbindung der Formel (1) eine Verbindung der nachstehenden Formel (1a) ist:

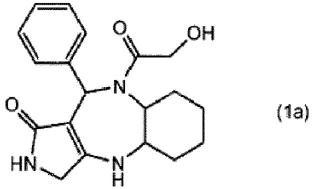

4. Verbindung zur Verwendung nach einem der Ansprüche 1 bis 3 oder ein Salz davon, wobei die Verbindung als pharmazeutische Zusammensetzung verabreicht wird, bei der es sich um Augentropfen handelt.

5. Verbindung zur Verwendung nach einem der Ansprüche 1 bis 4 oder ein Salz davon, wobei die Hornhautepithelstörung ein trockenes Auge ist.

6. Verbindung der Formel (1) oder ein Salz davon zur medizinischen Verwendung zur Unterdrückung von Hornhautepithelzellapoptose: 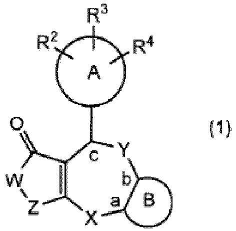 worin A ein Benzolring ist; R², R³ und R⁴, die gleich oder verschieden sein können, jeweils unabhängig Folgendes sind: ein Wasserstoffatom, ein Halogenatom, eine Hydroxygruppe, eine Alkylgruppe, eine Mercaptogruppe, eine Alkoxygruppen, eine Alkylthiogruppe, eine Alkylsulfonylgruppe, eine Acylgruppe, eine Acyloxygruppe, eine Aminogruppe, eine Alkylaminogruppe, eine Carboxygruppe, eine Alkoxycarbonylgruppe, eine Carbamoylgruppe, eine Nitrogruppe, eine Cyanogruppe, eine Trifluormethylgruppe, eine Alkenylgruppe, die gegebenenfalls einen Substituenten aufweist, eine Alkinylgruppe, die gegebenenfalls einen Substituenten aufweist, eine Arylgruppe, die gegebenenfalls einen Substituenten aufweist, eine Heteroarylgruppe, die gegebenenfalls einen Substituenten aufweist, eine Benzyloxygruppe, die gegebenenfalls einen Substituenten aufweist, eine Aryloxygruppe, die gegebenenfalls einen Substituenten aufweist, eine Heteroaryloxygruppe, die gegebenenfalls einen Substituenten aufweist, eine Arylaminogruppe, die gegebenenfalls einen Substituenten aufweist, eine Arylvinylgruppe, die gegebenenfalls einen Substituenten aufweist, oder eine Arylethinylgruppe, die gegebenenfalls einen Substituenten aufweist; B ein Cyclohexanring ist; X -NH- ist; Y -NR⁵- ist und Z -CH₂- ist (worin R⁵ eine Acylgruppe, die gegebenenfalls einen Substituenten aufweist, eine Niederalkylgruppe, die gegebenenfalls einen Substituenten aufweist, eine Alkoxycarbonylgruppe, die gegebenenfalls einen Substituenten aufweist, eine Carbamoylgruppe, die gegebenenfalls einen Substituenten aufweist, oder eine Sulfonylgruppe, die gegebenenfalls einen Substituenten aufweist, ist); W -NR¹- ist (worin R¹ ein Wasserstoffatom, eine Niederalkylgruppe, die gegebenenfalls einen Substituenten aufweist, oder eine Arylgruppe, die gegebenenfalls einen Substituenten aufweist, ist) und a, b und c jeweils die Position eines Kohlenstoffatoms anzeigen; mit der Maßgabe, dass:
(i) der optionale Substituent aus der aus folgenden bestehenden Gruppe ausgewählt ist: Halogenatom, Hydroxygruppe, Alkylgruppe, Mercaptogruppe, Alkoxygruppe, Alkylthiogruppe, Alkylsulfonylgruppe, Acylgruppe, Acyloxygruppe, Aminogruppe, Alkylaminogruppe, Carboxygruppe, Alkoxycarbonylgruppe, Carbamoylgruppe, Nitrogruppe, Cyanogruppe, Trifluormethylgruppe, Arylgruppe und Heteroarylgruppe.

## Revendications

1. Composé de formule (1) ou un sel de celui-ci, pour une utilisation dans le traitement d'un trouble de l'épithélium cornéen : 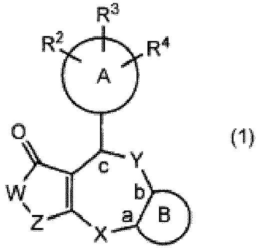 dans lequel A est un cycle benzène ; chacun de R², R³ et R⁴, qui peuvent être identiques ou différents, est indépendamment un atome d'hydrogène, un atome d'halogène, un groupe hydroxy, un groupe alkyle, un groupe mercapto, un groupe alcoxy, un groupe alkylthio, un groupe alkylsulfonyle, un groupe acyle, un groupe acyloxy, un groupe amino, un groupe alkylamino, un groupe carboxy, un groupe alcoxycarbonyle, un groupe carbamoyle, un groupe nitro, un groupe cyano, un groupe trifluorométhyle, un groupe alcényle qui peut porter un substituant, un groupe alcynyle qui peut porter un substituant, un groupe aryle qui peut porter un substituant, un groupe hétéroaryle qui peut porter un substituant, un groupe benzyloxy qui peut porter un substituant, un groupe aryloxy qui peut porter un substituant, un groupe hétéroaryloxy qui peut porter un substituant, un groupe arylamino qui peut porter un substituant, un groupe arylvinyle qui peut porter un substituant, ou un groupe aryléthynyle qui peut porter un substituant ; B est un cycle cyclohexane ; X est -NH- ; Y est -NR⁵- ; et Z est -CH₂- (où R⁵ est un groupe aryle qui peut porter un substituant, un groupe alkyle inférieur qui peut porter un substituant, un groupe alcoxycarbonyle qui peut porter un substituant, un groupe carbamoyle qui peut porter un substituant, ou un groupe sulfonyle qui peut porter un substituant) ; W est -NR¹- (où R¹ est un atome d'hydrogène, un groupe alkyle inférieur qui peut porter un substituant, ou un groupe aryle qui peut porter un substituant) ; et chacun de a, b et c représente la position d'un atome de carbone ; sous réserve que :
(i) ledit substituant optionnel soit choisi dans l'ensemble constitué par un atome d'halogène, un groupe hydroxy, un groupe alkyle, un groupe mercapto, un groupe alcoxy, un groupe alkylthio, un groupe alkylsulfonyle, un groupe acyle, un groupe acyloxy, un groupe amino, un groupe alkylamino, un groupe carboxy, un groupe alcoxycarbonyle, un groupe carbamoyle, un groupe nitro, un groupe cyano, un groupe trifluorométhyle, un groupe aryle, et un groupe hétéroaryle.

2. Composé pour une utilisation selon la revendication 1 ou un sel de celui-ci, dans lequel R¹ est H, et R⁵ est un groupe acyle qui peut porter un substituant.

3. Composé pour une utilisation selon l'une quelconque des revendications 1 et 2 ou un sel de celui-ci, dans lequel le composé de formule (1) est un composé de formule (1a) suivante :

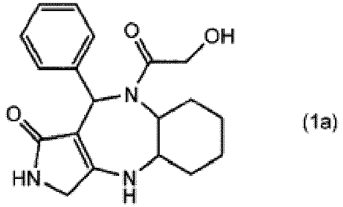

4. Composé pour une utilisation selon l'une quelconque des revendications 1 à 3 ou un sel de celui-ci, dans lequel le composé est administré sous la forme d'une composition pharmaceutique qui est une goutte oculaire.

5. Composé pour une utilisation selon l'une quelconque des revendications 1 à 4 ou un sel de celui-ci, dans lequel le trouble de l'épithélium cornéen est une sécheresse oculaire.

6. Composé de formule (1) ou un sel de celui-ci, pour un usage médical dans la suppression de l'apoptose de cellules épithéliales cornéennes : 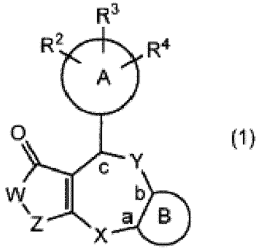 dans lequel A est un cycle benzène ; chacun de R², R³ et R⁴, qui peuvent être identiques ou différents, est indépendamment un atome d'hydrogène, un atome d'halogène, un groupe hydroxy, un groupe alkyle, un groupe mercapto, un groupe alcoxy, un groupe alkylthio, un groupe alkylsulfonyle, un groupe acyle, un groupe acyloxy, un groupe amino, un groupe alkylamino, un groupe carboxy, un groupe alcoxycarbonyle, un groupe carbamoyle, un groupe nitro, un groupe cyano, un groupe trifluorométhyle, un groupe alcényle qui peut porter un substituant, un groupe alcynyle qui peut porter un substituant, un groupe aryle qui peut porter un substituant, un groupe hétéroaryle qui peut porter un substituant, un groupe benzyloxy qui peut porter un substituant, un groupe aryloxy qui peut porter un substituant, un groupe hétéroaryloxy qui peut porter un substituant, un groupe arylamino qui peut porter un substituant, un groupe arylvinyle qui peut porter un substituant, ou un groupe aryléthynyle qui peut porter un substituant ; B est un cycle cyclohexane ; X est -NH- ; Y est -NR⁵- ; et Z est -CH₂- (où R⁵ est un groupe aryle qui peut porter un substituant, un groupe alkyle inférieur qui peut porter un substituant, un groupe alcoxycarbonyle qui peut porter un substituant, un groupe carbamoyle qui peut porter un substituant, ou un groupe sulfonyle qui peut porter un substituant) ; W est -NR¹- (où R¹ est un atome d'hydrogène, un groupe alkyle inférieur qui peut porter un substituant, ou un groupe aryle qui peut porter un substituant) ; et chacun de a, b et c représente la position d'un atome de carbone ; sous réserve que :
(i) ledit substituant optionnel soit choisi dans l'ensemble constitué par un atome d'halogène, un groupe hydroxy, un groupe alkyle, un groupe mercapto, un groupe alcoxy, un groupe alkylthio, un groupe alkylsulfonyle, un groupe acyle, un groupe acyloxy, un groupe amino, un groupe alkylamino, un groupe carboxy, un groupe alcoxycarbonyle, un groupe carbamoyle, un groupe nitro, un groupe cyano, un groupe trifluorométhyle, un groupe aryle, et un groupe hétéroaryle.
